# EUROPÄISCHE PATENTANMELDUNG

(11) **EP 1 442 707 A2**
(43) Veröffentlichungstag der Anmeldung: **04.08.2004**
(21) Anmeldenummer: 04004171.7
(22) Anmeldetag: 26.03.1996
(51) Int. Cl.: A61B 8/13, A61B 5/06, A61B 5/0456, G01S 15/89

(54) **Verfahren und Vorrichtung zur Generierung eines dreidimensionalen Bild-Datensatzes**

(30) Priorität: 08.03.1996 DE 19608971
(62) Teilanmeldung aus: 96104748.7
(71) Anmelder: TomTec Imaging Systems GmbH, 85716 Unterschleissheim (DE)
(72) Erfinder: Polz, Hans, Dr., 85456 Wartenberg (DE); Mumm, Bernhard, 82291 Mammendorf (DE)
(74) Vertreter: Müller, Frank Peter, Dipl.-Ing.

(57) **Zusammenfassung**

Bei einem Verfahren bzw. bei einer Vorrichtung zur Generierung eines diagnostisch verwertbaren dreidimensionalen Bilddatensatzes mit einem Ultraschallgerät wird der Ultraschallkopf zur Erzeugung einer Sequenz einer Vielzahl von Ultraschallbildern frei über ein zu untersuchende Volumen geführt. Zur Bestimmung der Positions- und Orientierungsdaten der jeweiligen Bildebenen ist ein elektromagnetisches Sensorsystem vorgesehen, dessen Empfänger mit dem Ultraschallkopf verbunden ist. Die Ultraschallbilder werden bei der Datenakquisition zunächst in einem Bildverarbeitungssystem als Rohdaten mit dem Positions- und Orientierungsdaten abgelegt. Diese Rohdaten werden dann in einem dreidimensionalen Datensatz transformiert, der das zu untersuchende Volumen erfaßt und in welchem sämtliche Bilddaten oder Bildwerte auf ein neues gemeinsames Bezugssystem, vorzugsweise auf ein kartesisches Koordinatensystem bezogen sind.

## Beschreibung

Die Erfindung bezieht sich auf ein Verfahren gemäß Oberbegriff Patentanspruch 1 sowie auf eine Vorrichtung zum Durchführen dieses Verfahrens gemäß Oberbegriff Patentanspruch 7.

Ein derartiges Verfahren bzw. eine derartige Vorrichtung sind grundsätzlich bekannt (US 41 00 916). Bei diesem bekannten Verfahren werden einzelne Ultraschall-Bilder unter Berücksichtigung der Positions- und Orientierungsdaten, die von einem mit Ultraschall arbeitenden Sensorsystem ermittelt werden, hinsichtlich ihrer Lage im Raum definiert. Ein dreidimensionaler, diagnostisch verwertbarer Datensatz, der eine tomographische Bilderfassung von einem zu untersuchenden Volumen ermöglicht, wird nicht generiert.

Der Erfindung liegt die Aufgabe zugrunde, eine Vorrichtung aufzuzeigen, mit der bei freier manueller Führung des Ultraschallwandlers eine tomografische Erfassung eines gesamten, zu untersuchenden Volumens oder dreidimensionalen Raumes durch einen dreidimensionalen Datensatz möglich ist.

Zur Lösung dieser Aufgabe ist ein Verfahren entsprechend dem kennzeichnenden Teil des Patentanspruches 1 ausgeführt und eine Vorrichtung entsprechend dem kennzeichnenden Teil des Patentanspruches 7 ausgebildet.

Bei der Erfindung wird bei freier Führung des Ultraschallwandlers entlang eines zu untersuchenden Volumens für eine tomographische Bilderfassung ein dreidimensionaler Datensatz generiert, der der tomografischen Bilderfassung entsprechend aus einer Vielzahl von Einzelbildem besteht, die das gesamte, zu untersuchende Volumen abdecken und sich ihrerseits aus einer Vielzahl von Bildwerten zusammensetzen.

Die Erfindung wird im folgenden anhand der Figuren an einem Ausführungsbeispiel näher erläutert. Es zeigen:
- Fig. 1: in schematischer Darstellung eine Ausführungsform der erfindungsgemäßen Vorrichtung zur Generierung von diagnostisch verwertbaren dreidimensionalen Datensätzen für eine tomografische Bilderfassung eines zu untersuchenden Volumens;
- Fig. 2: in schematischer Darstellung die jeweils im Bildverarbeitungssystem abgelegten Einzelbilder, zusammen mit graphischen Darstellungen des EKG-Zyklus und des Respirations-Zyklus eines Patienten;
- Fig. 3 - 5: ein Ablaufdiagramm der Arbeitsweise der erfindungsgemäßen Vorrichtung;
- Fig. 6: eine schematische Darstellung über die Transformation der Rohdaten in einen verwertbaren dreidimensionalen Datensatz.

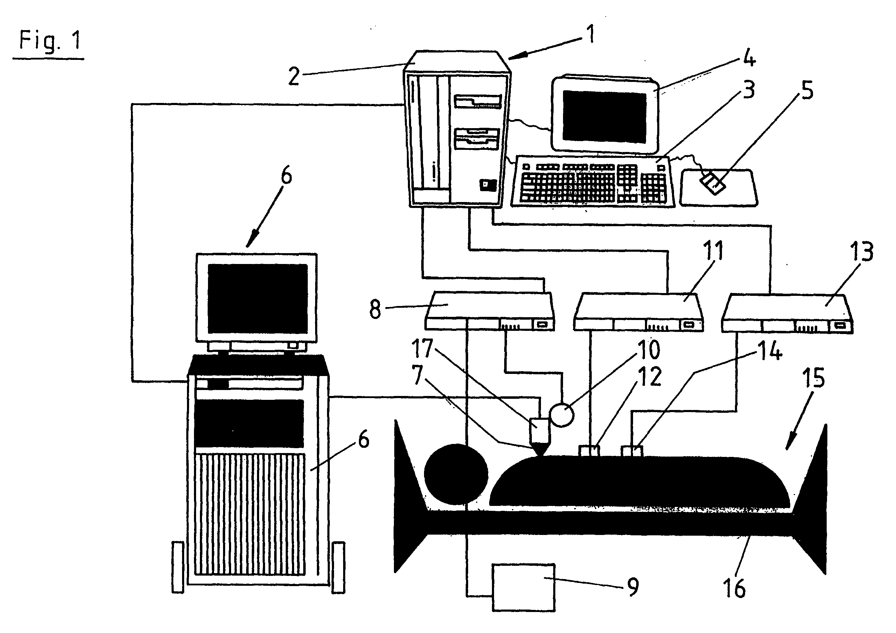

In der Fig. 1 ist 1 ein Bildverarbeitungssystem, welches im wesentlichen aus dem Rechner 2 und aus den an diesen angeschlossenen peripheren Geräten, wie Tastatur 3, Bildschirm 4 und Maussteuerung 5 usw. besteht.

Diesem Bildverarbeitungssystem sind zugeordnet:
- Ein Ultraschallgerät 6 mit Ultraschall-Kopf 7;
- ein elektromagnetisches Lage- bzw. Positionserfassungsgerät oder ein elektromagnetischer Sensor 8 mit einem Sender 9 und einem von Magnetspulen gebildeten Empfänger 10;
- ein EKG-Gerät 11 mit zugehörigen Elektroden 12;
- ein Gerät 13 zur Registrierung der Atemlage (Respirationszyklus) mit zugehörigen Sonden 14.

Mit 15 ist ein Patient bezeichnet, dessen Körper oder Körperbereich ein zu untersuchendes Volumen bildet. Der Patient 15 ist auf einer Liege 16 angeordnet.

Bei dem Ultraschallgerät 6 mit dem Ultraschallkopf 7 handelt es sich um ein dem Fachmann bekanntes und in der ärztlichen Diagnostik verwendetes Gerät zur Erzeugung von Ultraschall-Bildern, die jeweils einen Bereich des zu untersuchenden Volumens wiedergeben, der sich in der Bildebene des freigeführten Ultraschallkopfes 7 befindet. Am Ultraschallkopf 7, der von der untersuchenden Persan ( Arzt) manuell freigeführt wird, ist vorzugsweise ein Halter 17 vorgesehen, an welchem sich auch der Empfänger 10 des Sensorsystems 8 befindet.

Das System liefert aufgrund der von dem Sender 9 abgestrahlten Magnetfelder, die von dem Empfänger 10 bzw. den dortigen Spulen erfaßt werden, an seinem Ausgang Sensor-Daten (Positions- und Rotationsrohdaten), mit denen die Position und Orientierung des Empfängers 10 im Raum genau definiert ist, und zwar durch Translationsdaten in der X-, Y- und Z-Achse sowie durch Rotationsdaten um diese Achsen.

Ein solches elektromagnetisches Sensorsystem ist dem Fachmann bekannt, und zwar beispielsweise als ISOTRAK II der Firma POLHEMUS, One Hercules Drive, P.O.Box 560, Colchester, VT 05446.

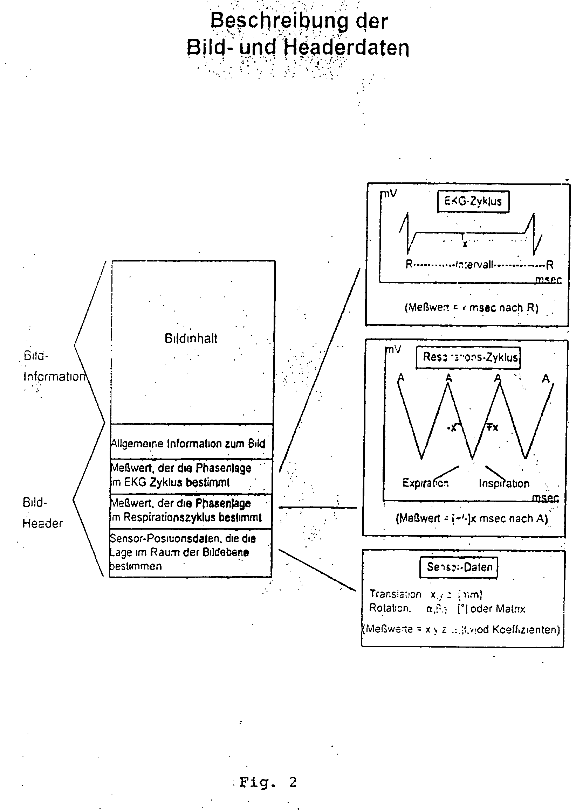

Das EKG-Gerät 11 ist ein solches, welches dem Fachmann aus der ärztlichen Praxis bzw. Diagnostik bekannt ist und welches in Abhängigkeit von dem EKG-Zyklus an einem Ausgang eine sich ändernde Spannung liefert, die im Diagramm a der Fig. 2 wiedergegeben ist und die typischen positiven und negativen R-Spitzen aufweist, zwischen denen jeweils ein R-Intervall gebildet ist. Bei dem Gerät 13 handelt es sich ebenfalls um ein dem Fachmann bekanntes Gerät, welches in der ärztlichen Praxis bzw. Diagnostik zur Erfassung des Respirationszyklus verwendet wird und in Abhängigkeit von diesem Repirationszyklus am Ausgang eine sich ändernde Spannung liefert, die im Diagramm b der Fig. 2 wiedergegeben ist.

Wie in der Fig. 2 auch wiedergegeben ist, wird im Bildverarbeitungssystem 1 in digitaler Form bei jedem Einzelbild zusätzlich zu dem Bildinhalt auch ein zugeordneter Bildheader abgelegt, der zusätzliche Informationen enthält, beispielsweise allgemeine Informationen zum Bild, einen Meßwert, der die jeweilige Phasenlage des Bildes im EKG-Zyklus bestimmt, einen Meßwert der die Phasenlage des jeweiligen Bildes in dem Respirationszyklus bestimmt sowie die Positions- und Orientierungsdaten, die die Lage der Bildebene des jeweiligen Einzelbildes im Raum bestimmen.

Um eine definierte Lage- bzw. Positionsbeziehung der jeweiligen Bildebene zu erreichen, wird das elektromagnetische Sensorsystem 8 verwendet, dessen Empfänger 10, der wenigstens eine Empfängerspule aufweist, an dem Halter 17 des freigeführten Schallwandlers oder Ultraschallkopfes 7 vorgesehen ist, und zwar in einer vorgegebenen räumlichen Beziehung aktiven Teil bzw. der Bildebene dieses Schallwandler.

Bei der Generierung der Positions- und Orientierungs-Daten (Translationdaten für X-, Y- und Z-Achse sowie Rotationdaten um diese Achsen) wird dann die räumliche Beziehung zwischen der Bildebene des Ultraschallkopfes 7 und dem Empfänger des Sensorsystems durch Kalibrierung, d.h. unter Verwendung von speziellen Kalibrier-Parametern errechnet. Hiermit ist es dann u.a. auch möglich, den Halter 17 mit dem Empfänger 10 an unterschiedlichen Ultraschall-Köpfen zu verwenden und diese jeweils durch unterschiedliche Kalibrierung oder Umrechnung der von dem Empfänger des Sensorsystems gelieferten Roh-Positionsdaten zu berücksichtigen.

Die Kalibrierung berücksichtigt somit die geometrische Lagebeziehung zwischen dem Empfänger und der Orientierung des aktiven Teils (Kristall) des Ultraschallkopfes 7 bzw. der Ebene (Bildebene), in der das vom Ultraschallkopf 7 erzeugte Bild liegt.

Bei der Erfassung der Bilder kommt es nicht auf die tatsächliche Lage der jeweiligen Bildebene im Raum an, sondern für die tomografische Bilderfassung reicht es aus, die relative Lage der einzelnen Bildebenen zueinander zu erfassen. Der Sender 9 des Sensorsystems 8 kann also im Umfeld der Vorrichtung beliebig positioniert werden.

Das Sensorsystem 8 errechnet also die Positions- und Orientierungswerte - definiert durch die drei Translationsfreiheitsgrade (X-, Y- und Z-Achse) und durch die drei Rotationsfreiheitsgrade - und ermittelt diese Daten an das Bildverarbeitungssystem, welche aus diesen Positions- und Orientierungsrohdaten durch Kalibrierung die echten Positions- und Orientierungsdaten errechnet, die dem jeweiligen Bildinhalt zugeordnet werden.

Die einzelnen Bilder werden von dem Ultraschallsystem 6 in einer vorgegebenen zeitlichen Folge (Sequenz) als analoge oder digitale Bild- oder Videosequenz an den Eingang des nachgeschalteten Bildverarbeitungssystems 1 geliefert. Mit dem Einschalten bzw. Aktivieren dieses Systems werden die Bilder, die beispielsweise Halbbilder oder Vollbilder sind, im Bildverarbeitungssystem 1 digitalisiert (bei analoger Videosequenz) oder aber in digitaler Form übernommen (bei digitaler Videosequeuz) und mit dem zugehörigen Bild-Header bzw. den Header-Daten digital in einem Speicher des Bildverarbeitungssystems 1 abgelegt (Bildeinzug).

Der Bildeinzug am Bildverarbeitungssystem 1 wird durch den Benutzer der Vorrichtung ausgelöst (z.B. durch Betätigen eines Fußschalters). Ebenso wird der Bildeinzug beispielsweise durch der Benutzer oder aber automatisch durch das Bildverarbeitungssystem 1 dann beendet, wenn der von der Vielzahl der Einzelbilder mit jeweils zugehörigem Header gebildete dreidimensionale Datensatz eine vorgegebene Größe erreicht hat.

Ist beispielsweise diese vorgegebene Größe 30 Megabyte, so bedeutet dies, daß bei einer Größe jedes Einzelbildes von 256 x 256 Pixel und bei einer Grauwerttiefe von 8 Bit sowie bei einem Einzug von 50 Halbbildern pro Sekunde, das Speichervolumen nach etwa 8 Sekunden und nach einem Einzug von 457 Eiuzelbildern gefüllt ist und der Bildeinzug dann von dem Bildverarbeitungsgerät automatisch beendet wird. Die Verwendung einer Bildmaskierung ermöglicht den Einzug größerer Bildmengen durch eine Reduktion des Speicherpaltzbedarfs pro Einzelbild.

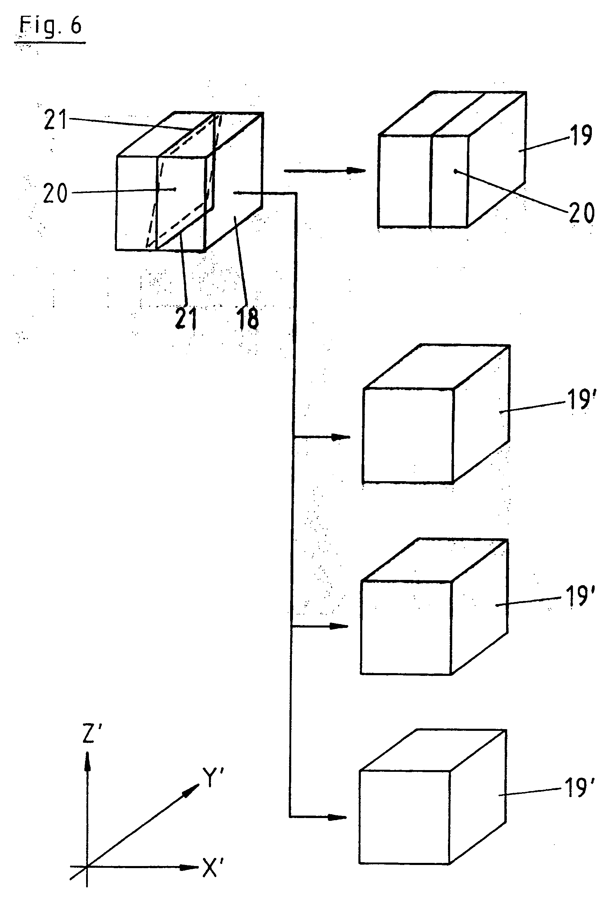

Da die Bildebenen der Einzelbilder durch die freie Führung des Ultraschallkopfes sehr unterschiedlich im Raum orientiert sind, erfolgt während des Bildeinzugs, d.h. in einer Datenerfassungs- oder akquisitionsphase zunächst eine Ablage der Einzelbilder als Rohdaten als Rohdatensatz 18 (Fig. 6). Nach Beendigung des Bildeinzugs erfolgt dann im Bildbearbeitungssystem 1 eine Transformation dieser Rohdaten unter Verwendung eines speziellen Programms auf ein gemeinsames Bezugssystem, beispielsweise kathesisches Koordinatensystem mit den Achsen X', Y' und Z' (Fig. 6), um so einen für die spätere Verwendung brauchbaren dreidimensionalen Datensatz 19 über das untersuchte Volumen zu erhalten.

Dieser dreidimensionale digitale Datensatz 19 ist grundsätzlich so aufgebaut, daß er eine Vielzahl von einzelnen Volumenelementen 20 (Voxel) enthält, die in diesem Datensatz 19 eine vorgegebene Lage in Bezug auf das gemeinsame Bezugssystem X'-, Y'- und Z'-Achse) aufweisen. Jeder Bildpunkt 20' der die Rohdaten 18 bildenden Bilder wird bei dieser Transformation in ein entsprechendes Voxel 20 dieses dreidimensionalen Datensatzes lagerichtig einsortiert. Ist bei der Bilderfassung ein bestimmter Bereich des zu untersuchenden Volumens nicht erfaßt worden, so kann bei der Tranformation auch dem entsprechenden Voxel 20 kein Bildwert zugeordnet werden, d.h. der dreidimensionale Datensatz 19 würde dann Lücken enthalten, die aber durch Interpolation ausgefüllt werden können.

Es besteht aber auch die Möglichkeit, daß bei der Bilderfassung bestimmte Bereiche des zu überprüfenden Volumens zufällig mehrfach abgetastet wurden und daß dann bestimmte Bildwerte 20' überbestimmt sind. In diesem Fall erfolgt entweder für überbestimmte Bildwerte im Bildverarbeitungssystem 1 die Bildung eines Durchschnittswertes oder aber es wird nach einer Auswahlroutine nur ein Bildwert weiter verwendet.

Die erfindungsgemäße Vorrichtung eignet sich in besonders vorteilhafter Weise auch zur tomografischen Bilderfassung von bewegten Organen, insbesondere von Organen, deren Bewegung in direktem Bezug zum Herzschlag steht (z.B. Herzgefäße), und zwar wiederum mit freier Führung des Ultraschall-Wandlers.

Hier besteht grundsätzlich die Möglichkeit, einen statischen dreidimensionalen Datensatz von einem solchen Gewebe zu generieren und hierbei geometrische Verzerrungen, die sich aus der Bewegung, beispielsweise Herztätigkeit ergeben, zu vermeiden. In diesem Fall erfolgt der Einzug der Bilder durch das Bildverarbeitungssystem 1 synchron mit dem Herzzyklus bzw. mit der positiven oder negativen R-Spitze des Herzyklus der zu untersuchenden Person. Der Herzzyklus bzw. die R-Spitze werden mittels eines EKG-Gerätes 11 ermittelt, an welches über Sonden 12 an den Patient 15 angeschlossen ist. Durch die Triggerung erfolgt jeder Bildeinzug in einem genau definierten Zeitfenter innerhalb des Herzzyklus, d.h. jeder Bildeinzug erfolgt jeweils in einem definierten zeitlichen Abstand (Phasenlage) nach dem Auftreten der jeweiligen, zur Triggerung verwendeten R-Spitze im EKG-Zyklus. Hiermit sind dann alle im Bildverarbeitungssystem 1 abgelegten und die Rohdaten bildenden Einzelbilder phasensynchron mit dem Herzzyklus, so daß diese Einzelbilder zu einem dreidimensionalen, statischen Datensatz 19 umgewandelt werden können, der bzw. dessen einzelne Voxel 20 keine geometrischen Verzerrungen aufweisen.

Durch Änderung der Phasenlage des Zeitfensters für den Bildeinzug in bezug auf die triggernde R-Spitze können dreidimensionale tomografische Datensätze 19 von dem untersuchten Volumen erstellt werden, die dieses Volumen jeweils zu unterschiedlichen Zeiten im EKG-Zyklus wiedergeben.

Mit der erfindungsgemäßen Vorrichtung lassen sich weiterhin auch dynamische, dreidimensionale Datensätze 19 von dem untersuchten Volumen, nämlich von bewegten Organen usw. herstellen. In diesem Fall erfolgt der Einzug der Bilder am Bildverarbeitungssystem nicht synchron mit dem Herzzyklus, sondern unabhängig von diesem. Auch hier wird wiederum im Bildheader jedes Einzelbildes 21 der Meßwert, der die Phasenlage im EKG-Zyklus bestimmt, mit abgelegt.

Dieser dreidimensionale, dynamische Datensatz 19, der wiederum durch Transformation der Rohdaten 18 auf ein gemeinsames Bezugssystem erhalten wird, besteht beispielsweise aus einer Vielzahl von Teil-Datensätzen 19', von denen jeder das gesamte zu untersuchende Volumen erfaßt und sich seinerseits aus einer Vielzahl von Volumenelementen 20 zusammensetzt, wobei die Teil-Datensätze 19' das untersuchte Volumen aber jeweils zu unterschiedlichen Zeitpunkten im EKG-Zyklus wiedergeben.

Die Erstellung des dynamischen, dreidimensionalen Datensatzes 19 erfolgt beispielsweise für einen kompletten EKG-Zyklus oder aber auch für nur einen Teil eines solchen Zyklus, wobei für die Generierung dieses Datensatzes 19 Bilder oder Bildwerte aus unterschiedlichen EKG-Zyklen verwendet werden, und zwar derart, daß nach oder bei der Transformation der Rohdaten 18 sämtliche vorhandenen Bildwerte aus unterschiedlichen EKG-Zyklen jeweils lagerichtig in die Voxel 20 des ihrer Phasenlage entsprechenden Teildatensatzes 19' eingeordnet werden.

Fehlen bei der Generierung der Teildatensätze 19' Bildwerte, weil bei der Bilderfassung Bereiche des untersuchten Volumens nicht erfaßt wurden, so können solche Lücken wiederum durch Interpolation von dem Bildverarbeitungssystem ausgefüllt. Sind mehrere Bildwerte pro Voxel 20 vorhanden, so wird diese Überbestimmung wiederum durch Bildung eines Durchschnittswertes oder durch Verwerfung von bestimmten Bildwerten oder durch Kombination beider Verfahren behoben.

Dreidimensionale Datensätze 19 können als statische oder dynamische Datensätze auch von Organen oder Körperteilen erstellt werden, die einer passiven Bewegung, beispielsweise durch Atmung unterworfen sind. Hierfür ist an das Bildverarbeitungssystem 1 das Gerät 13 angekoppelt, welches die Atemlage bzw. den Respirationszyklus des Patienten erfaßt. Dieser Respirationszyklus, der in der Fig. 2 im Diagramm b wiedergegeben ist, besteht jeweils aus der Expirations-Phase (Ausatmungsphase) und der Inspirations-Phase (Einatmungsphase). Mit diesem Respirationszyklus, beispielsweise mit den dortigen Spitzen A kann zur Erstellung eines durch die passive Bewegung unverzerrten statischen dreidimensionalen Datensatzes 19 das Bildverarbeitungssystem 1 wieder getriggert bzw. synchronisiert werden, und zwar derart, daß der Bildeinzug jeweils nur während eines vorbestimmten Fensters im Atemzyklus erfolgt. Grundsätzlich kann die Steuerung auch so erfolgen, daß der Bildeinzug nur dann erfolgt, wenn die Atemlage nicht aktiviert ist, also kein Respirationssignal vorliegt.

Grundsätzlich kann unter Berücksichtigung der Atemlage auch ein dynamischer, dreidimensionaler Datensatz generiert werden, wobei dann das Respirationszyklus in der Weise verwendet wird, wie dies vorstehend für den EKG-Zyklus bei der Erstellung des dynamischen Datensatzes 19 beschrieben wurde.

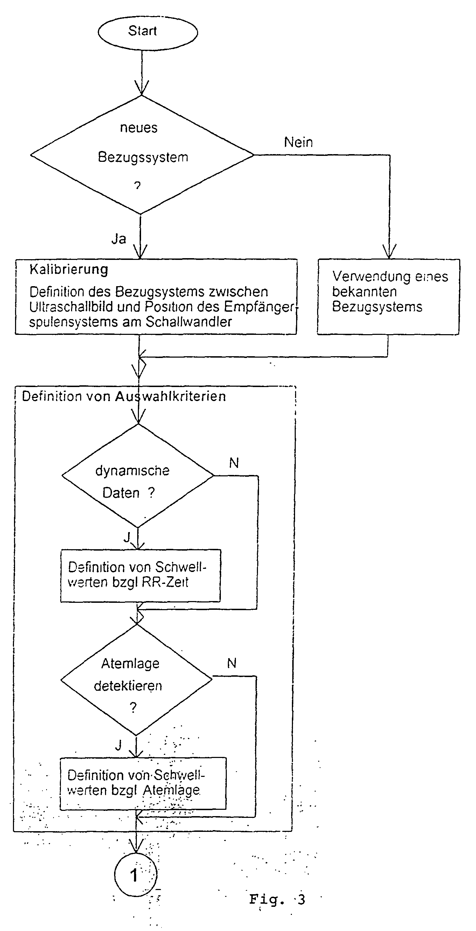

Die Fig. 3 - 5 zeigen in einem Ablaufdiagramm mehr im Detail die Arbeitsweise der erfindungsgemäßen Vorrichtung.

Die Fig. 3 betrifft die anfängliche Einstellung insbesondere des Bildverarbeitungssystems 1 vor der Erfassung der Bilder (Datenakquisition). Zunächst wird entschieden, ob das Bezugssystem, d.h. die räumliche Zuordnung zwischen der Bildebene des Ultraschallkopfes 7 und dem Empfänger 10 neu kalibriert werden muß, beispielsweise wegen Verwendung eines neuen Ultraschallkopfes 7, der im System noch nicht benutzt wurde.

Ist ein neues Bezugssystem notwendig, wird eine entsprechende Kalibrierung vorgenommen. Ist die Verwendung eines bekannten Bezugssystemes möglich, kann auf diese bzw. diese Kalibrierung zurückgegriffen werden. Im Anschluß daran erfolgt eine Definition von möglichen Auswahlkriterien. Soll eine dynamische Datenerfassung unter Berücksichtigung der Herztätigkeit erfolgen, so wird ein Schwellwert bezüglich der Größe des RR-Intervalls eingestellt. Diese Einstellung ist nicht notwendig, wenn eine dynamische Datenerfassung nicht gewünscht ist. Es erfolgt dann die Entscheidung, ob die Atemlage berücksichtigt werden soll. Ist dies der Fall, erfolgt eine Definition für den Schwellwert bezüglich der Atemlage, beispielsweise durch Einstellung eines maximalen und minimalen Schwellwertes für die Dauer eines Respiratonszyklus. Eine Definition des Schwellwertes entfällt, wenn die Atemlage nicht berücksichtigt werden soll.

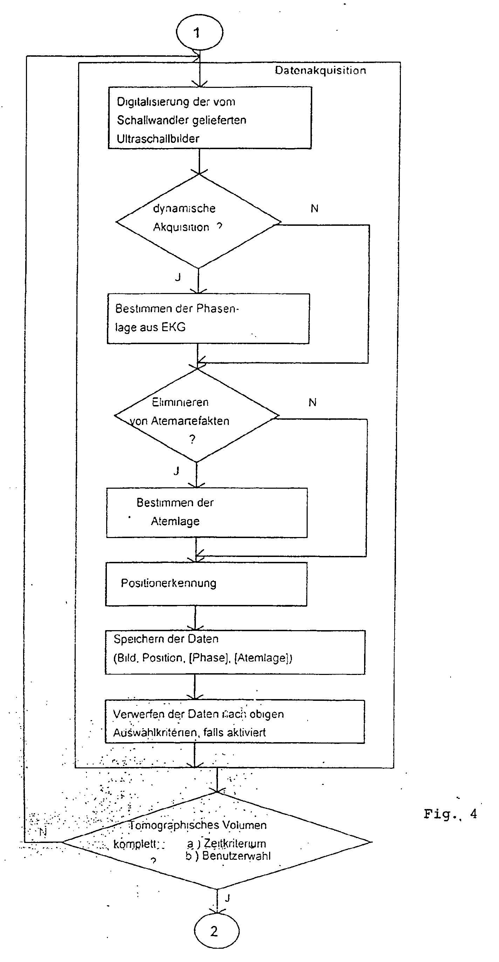

Nach dieser Voreinstellung erfolgt entsprechend Fig. 4 die Datenerfassung bzw. Akquisition. Zunächst werden die von dem Ultraschallkopf 7 gelieferten Ultraschallbilder digitalisiert. Dann wird bei einer dynamischen Datenerfassung die Phasenlage aus dem EKG-Zyklus und/oder die Phasenlage auf dem Respirationszyklus bestimmt, wobei diese Bestimmung entfällt, wenn eine dynamische Datenakquisition bzw. eine Datenakquisition unter Berücksichtigung der Atemlage nicht gewünscht sind.

In jedem Fall erfolgt bei jedem Bildeinzug die Positionserkennung. Die erfaßten Bilddaten werden dann zusammen mit den weiteren Daten als Rohdatensatz 18 abgelegt, wobei die weiteren Daten (Meßwert für die Phasenlage im EKG-Zyklus, Meßwert für die Phasenlage im Respirationszyklus und Positionsdaten) im Bildheader abgelegt werden.

Anschließend erfolgt eine Überprüfung der Daten anhand der im Verfahrensschritt der Fig. 3 getroffenen Auswahlkriterien. Solche Daten, die diesen Auswahlkriterien nicht entsprechen, werden verworfen und entfernt.

Die Datenakquisition ist beendet, wenn das zu überprüfende tomografische Volumen komplett ist, was entweder automatisch durch das Bildverarbeitungssystem nach einem Zeitkriterium festgestellt oder aber vom Benutzer bestätigt wird.

Sofern das tomografische Volumen nicht komplett ist, werden die Verfahrensschritte der Fig. 4 erneut durchgeführt.

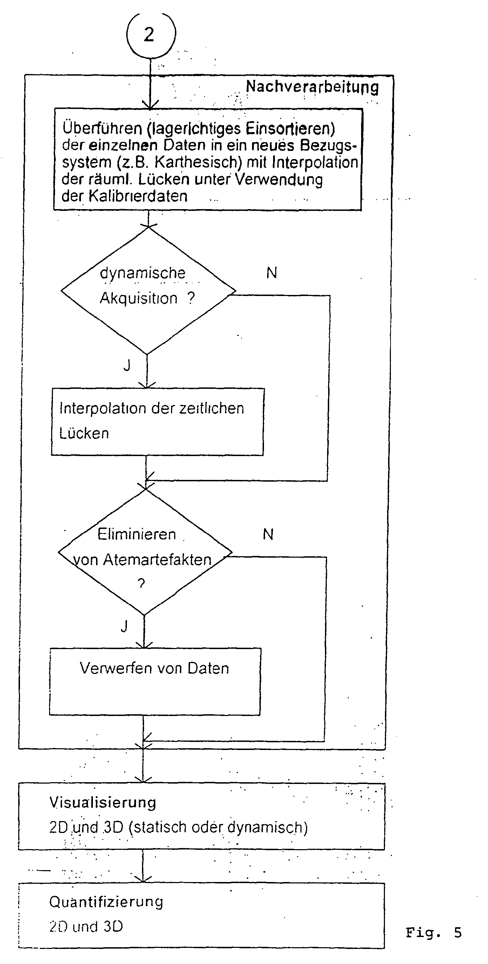

Die Fig. 5 zeigt die Verfahrensschritte für die Nachbearbeitung bzw. Transformation der Rohdaten 18 in den Datensatz 19. Es folgt zunächst das Überführen bzw. Transformieren (lagerichtiges Einsortieren) der einzelnen Bilddaten 20' in das neue, gemeinsame Bezugssystem, welches beispielsweise ein kartesisches Koordinatensystem ist. Weiterhin erfolgt das Ausfüllen von Lücken durch Interpolation. Diese Verfahrensschritte werden unter Verwendung der Kalibrierdaten durchgeführt.

Sofern eine dynamische Datenerfassung bzw. -Akquisition vorgenommen wird, erfolgt eine Interpolation von zeitlichen Lücken, insbesondere auch unter Verwendung von Bilddaten aus unterschiedlichen EKG-Zyklen. Erfolgte keine dynamiche Akquisition, so entfällt dieser Verfahrensschritt.

Anschließend erfolgt die Entscheidung, ob die Atemlage berücksichtigt werden soll oder nicht. Ist letzteres der Fall, so werden beispielsweise solche Daten eliminiert und verworfen, bei denen die Atemtätigkeit einen vorgegebenen Schwellwert übersteigt.

Die gewonnenen Daten werden schließlich so abgelegt, daß sie für eine zweidimensionale und/oder dreidimensionale Darstellung sowie auch für eine zweidimensionale und/oder dreidimensionale Quantifizierung verwendet werden können.

## Patentansprüche

1. Verfahren zur Generierung eines dreidimensionalen Bild-Datensatzes (19) eines bewegten Objekts, mittels
eines Ultraschallgerätes (6) zur Erzeugung einer Sequenz einer Vielzahl von Ultraschallbildern von dem zu untersuchenden Volumen des Objekts, und
eines an das Ultraschallgerät (6) angeschlossenen Bildverarbeitungssystems (1), dem die Sequenz der generierten Ultraschallbilder als Bilddaten zugeführt wird, durch Bestimmung der Position und der Orientierung des Ultraschallkopfes (7) und damit der räumlichen Lage des jeweils generierten Ultraschallbildes, wobei die Positions- und Orientierungsdaten eines jeden Ultraschallbildes an das Bildverarbeitungssystem (1) übertragen werden, das aus den Bilddaten und den Positions- und Orientierungsdaten den dreidimensionalen Bild-Datensatz (19) rekonstruiert, und wobei
zur Bestimmung der Position und der Orientierung des Ultraschallkopfes (7) ein elektromagnetisches System verwendet wird,
**dadurch gekennzeichnet,**
**dass** der Ultraschallkopf (7) manuell entlang des Objekts bewegt wird,
**dass** neben den Positions-, Orientierungs- und Bilddaten eines jeden Ultraschallbildes zusätzliche Bewegungsinformationen des Objekts generiert und als Bewegungsdaten an das Bildverarbeitungssystem (1) weitergeleitet werden,
**dass** die Generierung oder die Rekonstruktion der Bilddaten mittels der Bewegungsdaten gesteuert wird, und
**dass** einzelne Bewegungszustände des Objekts und/oder bewegte dreidimensionale Bilder rekonstruiert werden.

2. Verfahren nach Anspruch 1,
**dadurch gekennzeichnet,**
**dass** die Generierung oder die Rekonstruktion der Bilddaten mittels der Bewegungsdaten derart gesteuert wird, dass zu Zeiten grosser Bewegung des Objekts keine Bilddaten erzeugt oder rekonstruiert und zu Zeiten geringer Bewegung des Objekts die Bilddaten erzeugt oder rekonstruiert werden.

3. Verfahren nach Anspruch 1 oder 2,
**dadurch gekennzeichnet,**
**dass** ein an das Bildverarbeitungssystem (1) angeschlossenes EKG-Gerät einen EKG-Zyklus des Objekts definierende Bewegungssignale erzeugt, wobei das Bildverarbeitungssystem (1) Mittel aufweist, um den Einzug eines Ultraschall-Einzelbildes entsprechend einer bestimmten Phasenlage nach einem definierten Zeitpunkt des EKGZykus zu steuern.

4. Verfahren nach einem der vorhergehenden Ansprüche,
**dadurch gekennzeichnet,**
**dass** das Einziehen der Ultraschall-Bilder am Bildverarbeitungssystem (1) mittels der Bewegungssignale synchron mit dem EKG-Zyklus jeweils in einem vorgegebenen Zeitfenster dieses Zyklus erfolgt.

5. Verfahren nach einem der vorhergehenden Ansprüche,
**dadurch gekennzeichnet,**
**dass** das Einziehen der Ultraschall-Einzelbilder am Bildverarbeitungssystem (1) ständig erfolgt, und
**dass** die Bilddaten im Bildverarbeitungssystem anhand der Bewegungsdaten jeweils der Phasenlage im Herzzyklus entsprechend im dreidimensionalen Datensatz (19, 19) abgelegt werden.

6. Verfahren nach einem der vorhergehenden Ansprüche,
**dadurch gekennzeichnet,**
**daß** ein Gerät (13) zur Erfassung des Respirations-Zyklus verwendet wird, und daß ein Meßwert, der die Phasenlage im Respirationszyklus bestimmt, als Bewegungsdatum abgelegt wird.

7. Vorrichtung zur Generierung eines dreidimensionalen Bild-Datensatzes (19) eines bewegten Objekts, mit
einem Ultraschallgerät (6) zur Erzeugung einer Sequenz einer Vielzahl von Ultraschallbildern von dem zu untersuchenden Volumen des Objekts,
einem an das Ultraschallgerät (6) angeschlossenen Bildverarbeitungssystem (1), dem die Sequenz der generierten Ultraschallbilder als Bilddaten zugeführbar ist, und
mit einem elektromagnetischen Sensorsystem, das die Position und die Orientierung des Ultraschallkopfes (7) und damit die räumlichen Lage des jeweils generierten Ultraschallbildes bestimmt, wobei die Positions- und Orientierungsdaten eines jeden Ultraschallbildes an das Bildverarbeitungssystem (1) übertragbar sind, das aus den Bilddaten und den Positions- und Orientierungsdaten den dreidimensionalen Bild-Datensatz (19) rekonstruiert,
**dadurch gekennzeichnet,**
**dass** der Ultraschallkopf (7) manuell entlang des Objekts bewegbar ist,
**dass** ein Bewegungsgerät (11, 13) neben den Positions-, Orientierungs- und Bilddaten eines jeden Ultraschallbildes zusätzlich Bewegungsinformationen des Objekts ermittelt und als Bewegungsdaten an das Bildverarbeitungssystem (1) weiterleitet, und
**dass** die Generierung oder die Rekonstruktion der Bilddaten mittels der Bewegungsdaten steuerbar ist.

8. Vorrichtung nach Anspruch 7,
**dadurch gekennzeichnet,**
**dass** das elektromagnetische Sensorsystem einen Empfänger aufweist, der am Ultraschallkopf (7) oder an dem am Ultraschallkopf befestigten Halter (17) befestigt ist, und dass das Bildverarbeitungssystem (1) die von dem Sensorsystem gelieferten Positions- und Orientierungsdaten unter Verwendung von Parametern kalibriert, die den räumlichen Bezug zwischen dem Empfänger (11) des Sensorsystems und dessen Orientierung im Raum und der Lage der Bildebene des Ultraschallkopfes (7) berücksichtigen.

9. Vorrichtung nach einem der Ansprüche 7 oder 8,
**dadurch gekennzeichnet**,
das das Bewegungsgerät ein EKG-Gerät (11) oder ein Gerät (13) zur Erfassung der Respiration eines Menschen ist.
